# EUROPEAN PATENT APPLICATION

(11) **EP 0 629 857 A2**
(43) Date of publication of application: **21.12.1994**
(21) Application number: 94304201.0
(22) Date of filing: 10.06.1994
(51) Int. Cl.: G01N 33/543, G01N 33/563, G01N 33/53

(54) **Immunoassay for quantitatively determining antigens**

(30) Priority: 16.06.1993 JP 144640/93
(71) Applicant: NISSHIN FLOUR MILLING CO., LTD., Chuo-ku, Tokyo 103 (JP)
(72) Inventor: Imai, Shinjirou, c/o Nisshin Flour Milling Co. Ltd, Ohimachi, Iruma-gun, Saitama-ken (JP); Sakai, Hiroshi, c/o Nisshin Flour Milling Co. Ltd, Ohimachi, Iruma-gun, Saitama-ken (JP); Sato, Takeya, c/o Nisshin Flour Milling Co., Ltd., Chuo-ku, Tokyo (JP); Sasaki, Kazuyuki, c/o Nisshin Flour Milling Co.Ltd, Ohimachi, Iruma-gun, Saitama-ken (JP)
(74) Representative: Marshall, Monica Anne

(57) **Abstract**

An immunoassay for quantitatively determining an antigen in a sample is disclosed, which comprises immobilizing a primary antibody in a purified state or in a diluted serum to the surface of a carrier via an affinity ligand, particularly protein A, immobilizing an antigen to be determined to the primary antibody, and reacting a labelled secondary antibody with the antigen. This method can attain a markedly improved identification limit and measurement limit which have not been attained by conventional methods, eliminates purification of the primary antibody, and enables one to save the amount of immobilized antibody. In addition, it provides a measuring method which is inexpensive and yet effective and which can determine a trace amount of ingredient with high sensitivity.

## Description

### FIELD OF THE INVENTION

This invention relates to an immunoassay for quantitatively determining antigens in a sample.

### BACKGROUND OF THE INVENTION

Immunoassay useful for determinig trace amounts of substances have been extensively employed in various fields including basic investigations and clinical diagnosis.

In the non-competitive immunoassay system well known as the sandwitch method, antigens to be determined are captured on a solid phase via a primary antibody immobilized on the solid phase, then the antigens are reacted with a secondary antibody labeled with a proper marker such as an enzyme or radioactive iodine, followed by measuring the activity of the bound marker. Thus, the antigens are quantitatively determined based on the measured activity.

The most popularly employed method for immobilizing a primary antibody is a method which comprises applying an antibody-containing solution to a microtiter plate made of a synthetic resin such as polystyrene to thereby immobilize the antibody onto the plate through physical adsorption or formation of chemical bonds. In immobilizing antibodies by such method, the antibody molecules are considered to be adsorbed or bound disorderly onto the surface of the carrier through intermolecular attraction with the carrier or through formation of covalent bonds. Hence, the variable region of the antibody which should be used for specifically binding an antigen may be bound to the carrier, that is, all of the antigen-binding region of the immobilized antibody molecules may not effectively bind the antigen, which means decrease of the antigen-binding ability, or titer, of the immobilized antibody. This results in a lower sensitivity than is desired.

In determining trace amounts of substances according to the sandwitch method, the primary antibody must usually be purified before use, because antiserum contains large amounts of other proteins such as albumin than immunoglobulin, which compete against immobilization of immunoglobulin. If immobilization of a primary antibody can be conducted using antiserum, complicated purification of immunoglobulin can be eliminated. Elimination of the purification serves to save an expensive antiserum. In addition, antiserum generally has greater stability than that of purified immunoglobulin.

As described above, conventional immobilizing methods are not fully satisfactory, and there it has been desired to develop a system in which a primary antibody is immobilized onto a carrier in high yield, and most of the antigen-binding region of the antibody is in a state of being bindable with an antigen.

### SUMMARY OF THE INVENTION

Under such circumstances, the inventors have made intensive investigations to enhance the efficiency of the binding reaction between an employed antibody and an antigen for solving the above-described problem with the prior art and, as a result, have found a method for determining trace amounts of substances with low cost and high efficiency and sensitivity not having been attained by the conventional immobilizing methods, thus having completed the present invention.

The present invention provides an immunoassay for quantitatively determining an antigen, which comprises immobilizing a primary antibody onto the surface of a carrier via an affinity ligand, allowing an antigen in question to be bound to the immobilized primary antibody, reacting a labeled secondary antibody with the antigen, and measuring the labeled secondary antibody. This method provides enhanced identification limit and determination limit with respect to the non-competitive, immunologically determining method, and sufficiently satisfies the demand that the necessary amount of an immobilized antibody be decreased.

In addition, the method of the present invention permits to use not only purified IgG but non-purified IgG in a state of antiserum, i.e., to react antiserum with an affinity ligand immobilized on the surface of a carrier to thereby immobilize IgG contained in the serum. In the case of using antiserum, the amount of the primary antibody can be decreased and, in addition, complicated procedures for purifying immunoglobulin can be eliminated.

### BRIEF DESCRIPTION OF DRAWINGS

Fig. 1 is a graph showing the correlation between amount of antigen and absorbance measured by the conventional method.

Fig. 2 is a graph showing the correlation between amount of antigen and absorbance measured by the method of the present invention.

Fig. 3 is a graph showing the correlation between amount of antigen and absorbance measured by the method of the present invention.

### DETAILED DESCRIPTION OF THE INVENTION

In accordance with the present invention, the antigens to be determined include hormones such as growth hormone, insulin, adrenocorticotropic hormone (ACTH) , thyroid-stimulating hormone (TSH), luteinizing hormone (LH) and gut hormone, e.g., glicentin; growth factors such as epidermal growth factor (EGF), nerve growth factor (NGF), platelet-derived growth factor (PDGF), fibroblast growth factor (FGF), insulin-like growth factor (IGF) and hepatosite growth factor; bacterial toxins; bacterial metabolites and antibodies thereto; exosporium components; virus capsid components; enzymes such as alkaline phosphatase (ALP), glutamate-oxaloacetate transaminase (GOT), glutamate-pyruvate transaminase (GPT), lactate dehydrogenase (LDH), blood clotting factor, RNA polymerase and DNA polymerase; lipoproteins such as very low-density lipoprotein (VLDL), high-density lipoprotein (HDL) and low-density lipoprotein (LDL); receptors such as hormone receptors, e.g., insulin receptor, growth hormone receptor, EGF receptor and nerve receptor, e.g., acetylcholine receptor; cancer markers such as α-fetoprotein (AFP), ferritin and carcinoembryonic antigen (CEA); cell surface antigens such as histocompatibility antigen; autoantibodies; c-reactive proteins; and other physiological active substances such as endothelin, peptide enzyme inhibitors, e.g., pancreatic trypsin inhibitor, amylase inhibitor, α₂ macroglobulin; and complements such as carrier protein, IGF-binding protein and transferrin.

As the affinity ligand in the present invention, there may be used any substances being capable of binding the Fc part in the antibodies, which include e.g. protein A, protein G, anti-gamma IgG antibody, etc. In the preferred embodiments of the present invention, protein A is preferably used. In addition, affinity carriers for purifying immunoglobulin such as protein A-Sepharose (trade name of Pharmacia Co.), SPA beads to be used as scintillator for enhancing efficiency of radiation energy to luminescence in radioimmunoassay such as scintillation proximity assay protein A reagent (trade name of Amersham Co., Ltd., England) and the like may also be used.

Several embodiments of the present invention are illustrated below for describing the present invention in more detail.

Protein A is adsorbed or chemically bound to a microtiter plate, beads or the like. There exist no limitations as to this procedure, but usually a protein A solution at a concentration of 2 - 100 µg/ml is brought into contact with the plate, beads, or the like at 0 - 37°C for a period of 10 minutes to 50 hours. Non-adsorbed or non-bound protein A is removed by well washing the plate, beads or the like with a buffer solution, then a solution of suitable blocking reagents such as bovine serum albumin is added thereto to prevent non-specific adsorption of an antigen or a secondary antibody to be added in the subsequent step. This procedure may be conducted according to the manner employed in a general immunoassay by, for example, adding thereto a solution of bovine serum albumin in a neutral phosphate buffer solution, followed by allowing to stand the resulting system. After removing the blocking reagent by washing, a primary antibody (IgG) specifically binding a substance to be tested or an antiserum containing the primary antibody is added to the system and, after keeping the system at 0 - 37°C for a period of 10 minutes to 20 hours, the system is well washed with a buffer solution. As a result of this procedure, IgG of the antibody added is effectively captivated by protein A having been immobilized to the plate or beads, thus immobilization of the primary antibody being completed.

After completion of immobilization of the primary antibody, a solution containing a substance to be tested is added to the system, and the resulting system is kept at O - 37°C for a period of 10 minutes to 50 hours to thereby bind the substance to be tested to the primary antibody. Subsequent operation may be conducted in accordance with the procedure of immunoassay generally employed in the sandwitch method. For instance, an antibody whose Fab' part has been labeled with an enzyme such as horseradish peroxidase (HRP) or a marker such as radioactive iodine or biotin is added thereto as a secondary antibody, and the activity of immobilized marker or the like is measured in an ordinary manner. Needless to say, it is also possible to use a third antibody which is directed against the secondary antibody and labeled with a suitable marker, instead of using the labeled secondary antibody.

Below are mentioned remarkable advantages achieved by the method of the present invention.
1) Since the immobilized antibody molecules are essentially bound to protein A via the constant region which is not directly related to the formation of bonds with antigens, the antigen-recognizing site in the antibody molecules is not consumed for the formation of bonds with a carrier.
2) Since antibody molecules quantitatively bind protein A, it suffices to add the antibody in an amount corresponding to the immobilized protein A. Accordingly it is not necessary to add excess amount of the antibody unlike the conventional methods.
3) Since antibody molecules specifically bind protein A, immobilization can be conducted without purifying the antibody as is different from the conventional non-specific immobilization, which serves to eliminate the complicated purification procedures and save antibody.

As a result of the immobilization attained as in 1), the primary antibody can be bound to the surface of a carrier with a definite space from the surface due to the presence of protein A therebetween, which serves to markedly decrease steric hindrance by the carrier.

In addition, the specific binding between protein A and the antibody is not seriously interfered by other proteins existing in serum. Hence, the antibody can react with protein A in a form of serum.

The decreased steric hindrance serves to enhance reactivity of the antibody with an antigen, which leads to enhanced sensitivity. Thus, the method of the present invention provides a high level of identification limit which could not be attained by the prior art and enables one to reduce the necessary amount of immobilized antibody and eliminate the procedure of purifying the primary antibody.

The present invention is further illustrated by the following examples in which there were used solutions and reagents of the following formulations.
PBS: 10 mM sodium phosphate buffer solution (pH 7.2), 0.15 M sodium chloride;
PB·Tween:
PBS + 0.05% Tween 20
EIA-Buffer solution:
PBS + 10% Block Ace (trade name of Meiji Milk Products Co., Ltd.) + 0.015% Triton X-305
PBS·BSA:
PBS + 0.1% Bovine serum albumin
Blocking solution:
PBS + 25% Block Ace
Color-forming solution:
To 10 ml of a potassium citrate buffer solution (0.1 M, pH 4.5) containing o-phenylenediamine (1 mg/ml) is added, upon use, 4 µl of a 30% hydrogen peroxide solution.

### EXAMPLE 1

Preparation of a plate having immobilized thereon protein A

50 µl of a protein A solution (10 µg/ml, dissolved in PBS) was dispensed into each well of a 96-well Nunc immunoplate MaxiSorp (trade name of Nunc Co., Ltd., Denmark), and the immunoplate was allowed to stand overnight at 4°C to adsorb protein A onto the immunoplate. The protein A solution in the wells was removed, and the inside of each well was washed three times with PBS using a plate washer (made by Bio-Teck Co., model EL403). 300 µl of the blocking solution was added to each well and, after keeping the immunoplate at 4°C for 16 hours, the inside of each well was well washed with PBS to complete the preparation of a plate having fixed thereon protein A.

### EXAMPLE 2

Immobilization of primary antibody

50 µl of a solution of anti-endothelin (15-21) rabbit IgG (product of Immuno-Biological Laboratories Co., Ltd.; 5 µg/ml dissolved in EIA buffer solution) was added to each well of the plate having fixed thereon protein A and, after keeping the plate at 25°C for 2 hours, each well was washed three times with PBS to complete the immobilization of the primary antibody. Additionally, as a control experiment, the primary antibody was directly immobilized onto the immunoplate in the conventional manner as follows. That is, 50 µl of the anti-endothelin solution (5 µg/ml dissolved in PBS) was added to each well of 96-well immunoplate MaxiSorp, and the immunoplate was kept at 4°C for 16 hours, followed by washing three times with PBS to complete the immobilization of the primary antibody.

### EXAMPLE 3

Precision of measurement of endothelin

To each well of the two kinds of plates having immobilized thereon the primary antibody as prepared in Example 2 was added 100 µl of a PBS solution containing endothelin-1 in a concentration of 1 to 30 pg/ml, and the plates were kept at 25°C for two hours under shaking. After washing three times with PBS·Tween, 50 µl of an EIA buffer solution containing 0.6 µg/ml of a labeled secondary antibody of anti-endothelin-1 rabbit IgG·Fab'·HRP (product of Immuno-Biological Laboratories Co., Ltd.) was added to each well, and the plate was kept at 25°C for 2 hours under shaking. After washing five times with PBS·Tween, 100 µl of the coloring solution was added to each well and, after keeping the plate at 25°C for 30 minutes under shaking, 100 µl of 2 N sulfuric acid was added to each well, followed by measuring absorbance (A₄₉₀₋₆₅₀) using a plate reader (made by Molecular Device Co.; model M-Tmax). Tables 1 and 2 show that the method of the present invention provides excellent quantitativeness. In comparison with the conventional method, the method of the present invention provides larger difference in absorbance for different amounts of antigen and less variation between the wells. This may be attributed to efficient utilization and stabilization of the primary antibody owing to protein A.

**Table 1**

| Conventional Method | | | | |
|---|---|---|---|---|
| Amount of Endothelin (pg/well) | Absorbance | Average Absorbance | Standard Deviation | Coefficient of Variation |
| 0 | 0.009 | | | |
| 0 | 0.010 | 0.010 | 0.001 | 9.667 |
| 0 | 0.001 | | | |
| 0.1 | 0.013 | | | |
| 0.1 | 0.013 | 0.014 | 0.002 | 12.08 |
| 0.1 | 0.016 | | | |
| 0.3 | 0.021 | | | |
| 0.3 | 0.024 | 0.022 | 0.003 | 11.62 |
| 0.3 | 0.019 | | | |
| 1.0 | 0.037 | | | |
| 1.0 | 0.051 | 0.042 | 0.008 | 19.25 |
| 1.0 | 0.037 | | | |
| 3.0 | 0.087 | | | |
| 3.0 | 0.082 | 0.086 | 0.003 | 3.370 |
| 3.0 | 0.087 | | | |

**Table 2**

| Method Using Protein A | | | | |
|---|---|---|---|---|
| Amount of Endothelin (pg/well) | Absorbance | Average Absorbance | Standard Deviation | Coefficient of Variation |
| 0 | 0.087 | | | |
| 0 | 0.082 | 0.084 | 0.003 | 3.537 |
| 0 | 0.082 | | | |
| 0.1 | 0.087 | | | |
| 0.1 | 0.089 | 0.089 | 0.001 | 1.302 |
| 0.1 | 0.089 | | | |
| 0.3 | 0.103 | | | |
| 0.3 | 0.105 | 0.105 | 0.001 | 1.103 |
| 0.3 | 0.105 | | | |
| 1.0 | 0.164 | | | |
| 1.0 | 0.168 | 0.167 | 0.002 | 1.249 |
| 1.0 | 0.167 | | | |
| 3.0 | 0.310 | | | |
| 3.0 | 0.322 | 0.319 | 0.008 | 2.374 |
| 3.0 | 0.324 | | | |

### EXAMPLE 4

Example of determining endothelin in plasma

Endothelin-1 was added to rat plasma to a concentration of 10 or 30 pg/ml. Acetic acid was added to the plasma to a concentration of 4%, and then subjected to Sep-Pak C₁₈ column (trade name of Millipore Co.) which has been pretreated with, successive, a 4% acetic acid - 86% ethanol mixed solution and methanol-distilled water. After washing the column with distilled water, elution was conducted using a 4% acetic acid - 86% ethanol mixed solution. The eluate was evaporated to dryness under reduced pressure and dissolved in an EIA buffer solution. The resulting solution was used for determining endothelin in the same manner as in Example 3. The amount of endothelin in the plasma was determined based on calibration curve for known concentrations of endothelin solutions. It is apparant that, in comparison with the conventional method, the method of the present invention provides more accurate data for the amounts of added endothelin with low variation between wells (Tables 3 and 4). This indicates that the method of the present invention decreases adverse influence of measurement-inhibiting factors derived from the plasma and is extremely effective for application to the field of clinical inspections.

**Table 3**

| Determination of Endothelin (10 pg/ml) in Rat Plasma | | | | |
|---|---|---|---|---|
| Well No. | Calculated Concentration (pg/ml) | Average Concentration (pg/ml) | Standard Deviation | Coefficient of Variation |
| Conventional Method | | | | |
| 1 | 7.579 | | | |
| 2 | 8.850 | 8.533 | 0.635 | 7.447 |
| 3 | 8.850 | | | |
| 4 | 8.850 | | | |

| Inventive Method Using Protein A | | | | |
|---|---|---|---|---|
| 1 | 9.995 | | | |
| 2 | 10.84 | 10.63 | 0.478 | 4.503 |
| 3 | 11.12 | | | |
| 4 | 10.56 | | | |

**Table 4**

| Determination of Endothelin (30 pg/ml) in Rat Plasma | | | | |
|---|---|---|---|---|
| Well No. | Calculated Concentration (pg/ml) | Average Concentration (pg/ml) | Standard Deviation | Coefficient of Variation |
| Conventional Method | | | | |
| 1 | 27.49 | | | |
| 2 | 31.72 | 29.08 | 1.842 | 6.336 |
| 3 | 28.76 | | | |
| 4 | 28.34 | | | |

| Inventive Method Using Protein A | | | | |
|---|---|---|---|---|
| 1 | 31.85 | | | |
| 2 | 31.85 | 32.06 | 0.420 | 1.311 |
| 3 | 32.69 | | | |
| 4 | 31.85 | | | |

### EXAMPLE 5

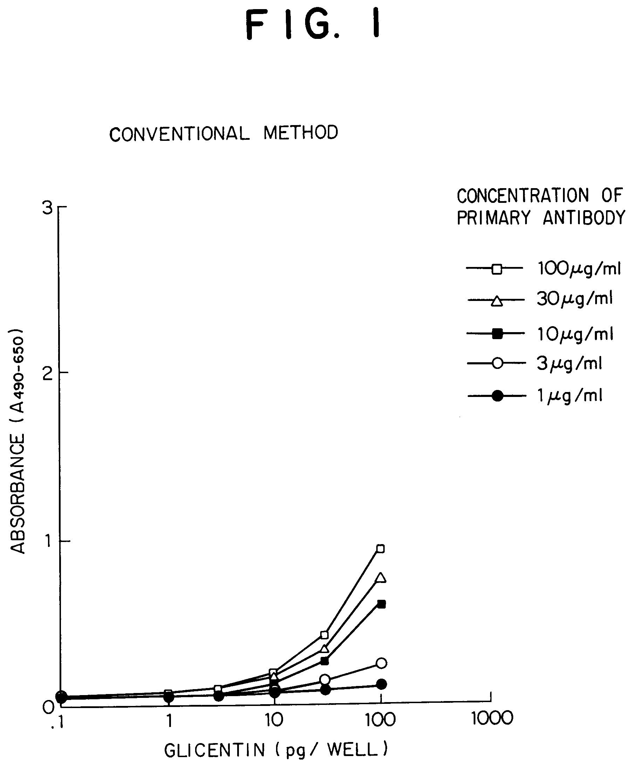

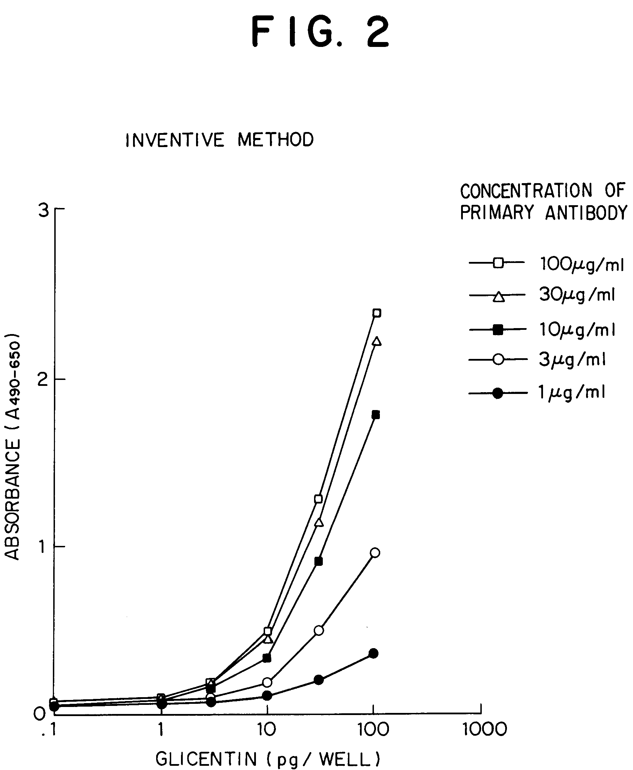

50 µl of a solution of anti-glicentin (49-69) rabbit IgG (1, 3, 10, 30 or 100 µg/ml dissolved in PBS·BSA) was added to each well of a plate having fixed thereon protein A and prepared according to the process described in Example 1 and, after keeping at 25°C for 2 hours, each well was washed three times with PBS to immobilize the primary antibody. As a control experiment using a conventional method, 50 µl of a solution of the anti-glicentin antibody in PBS in varying concentration was added to each well of a 96-well immunoplate MaxiSorp to immobilize the antibody in a similar way as in Example 2. The anti-glicentin (49-69) rabbit IgG used herein was prepared by chemically synthesizing a peptide corresponding to the amino acid sequence of 49th to 69th amino acid units of glicentin from N-termius, allowing the peptide to adsorb on 50% polyvinyl pyrrolidone, immunizing rabbits with the peptide together with Freund's complete adjuvant, purifying the resulting antiserum using Ampure™ PA kit (trade name of Amersham Co., Ltd., England). 100 µl of a solution of glicentin (10, 30, 100, 300, 1000 pg/ml dissolved in PBS·BSA) was added to each well of the plates having immobilized thereon various amounts of the primary antibody, and the plates were kept at 25°C for 3 hours under shaking. After washing five times with PBS, 50 µl of a solution of a labeled secondary antibody of anti-glicentin (1-32) rabbit IgG·Fab'·HRP (2.2 µg/ml dissolved in PBS·BSA)(prepared according to the description of "Proceedings of The Thirteenth Gut Hormone Conference", edited by JAPANESE SOCIETY OF GUT HORMONES and published by IGAKU TOSHO SHUPPAN LTD. in 1992, Vol. 11, pp. 358-363) was added to each well, and the plates were kept at 25°C for 2 hours. Subsequent procedures were conducted in the same manner as in Example 3 to measure absorbance. The results thus obtained are shown in Figs. 1 and 2. In comparison with the conventional method, the method of the present invention provides larger change in absorbance for different amounts of antigen, and this effect is also remarkable when a low concentration of antibody solution is used. That is, a change in absorbance not having been obtained by the conventional method even when using 100 µg/ml of the primary antibody can be obtained by the method of the present invention using the antibody in a concentration as low as 3 µg/ml. In addition, identification limit of the antibody according to the method of the present invention is as small as 2 pg, which has been about 10 pg with respect to the conventional method, thus measuring sensitivity being significantly enhanced.

### EXAMPLE 6

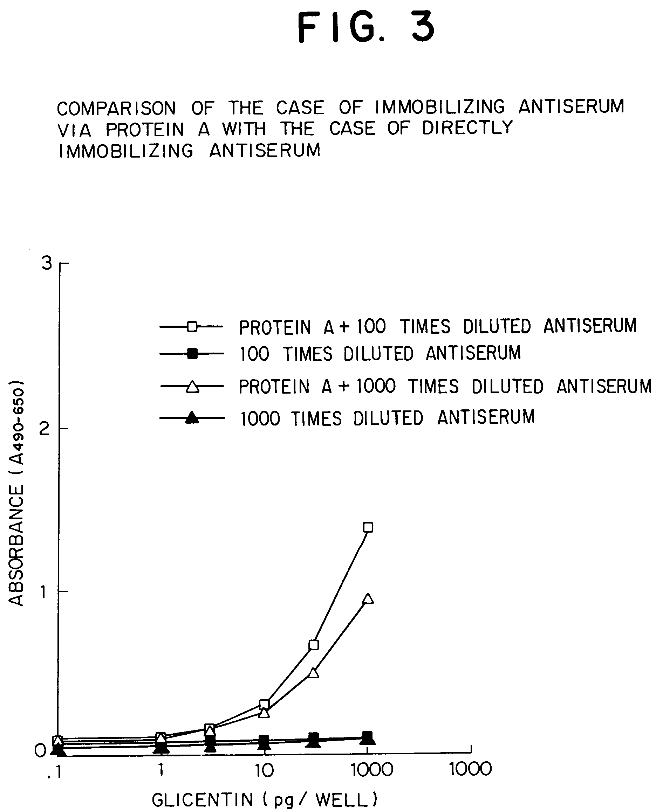

50 µl of a solution of anti-glicentin (49-69) antiserum (diluted 100 times or 1000 times with PBS·BSA) was added to each well of a plate having fixed thereon protein A as prepared according to the process described in Example 1 and, after keeping at 25°C for 2 hours, each well was washed three times with PBS to immobilize the primary antibody. As a control experiment, 50 µl of a solution of the above-described antiserum (diluted 100 times or 1000 times with PBS) was added to each well of a 96-well immunoplate MaxiSorp and, after keeping at 4°C for 16 hours, the plate was washed three times with PBS to complete the immobilization. The antiserum used was the antiserum for preparing the primary antibody used in Example 5. 100 µl of a solution of glicentin (10, 30, 100, 300 or 1000 pg/ml dissolved in PBS·BSA) was added to each well of the thus prepared plates, and the plates were kept at 25°C for 3 hours under shaking. After washing five times with PBS, 50 µl of a solution of a labeled secondary antibody of anti-glicentin (1-32) rabbit IgG Fab'·HRP (2.2 µg/ml dissolved in PBS·BSA) used in Example 5 was added to each well, and the plates were kept at 25°C for 2 hours. Subsequent procedures were conducted in the same manner as in Example 3 to measure the absorbance. The results are shown in Fig. 3. In the method of the present invention, even a primary antibody is in a form of serum can provide large change in absorbance for different amounts of antigen, thus the method being sufficiently practical. In contrast, in the case of directly immobilizing the antiserum as in the control experiment, there appeared almost no difference in absorbance for different amounts of the antigen, thus determination of the antigen being impossible. IgG level in rabbit antiserum is said to be usually about 10 mg/ml and, as to the amount of IgG used in Example 5, 5.5 mg of IgG was obtained by purification from 1 ml of the serum. Accurate IgG concentration of the antiserum used in this experiment was not known but, when the above-described estimated IgG concentration of 10 µg/ml is employed, the antiserum diluted 1000 times corresponds to 10 µg/ml, and the antiserum diluted 100 times corresponds to 100 µg/ml. The results of this experiment using antiserum show that the change in absorbance is significantly larger than in the control experiment in Example 5 wherein purified IgG was directly immobilized. In addition, concentration of the antibody in the serum diluted 1000 times is calculated to be 5.5 µg/ml based on the purified yield of IgG described above. Since the primary antibody diluted 1000 times used in this experiment shows substantially the same change in absorbance as the primary antibody solution at a concentration of 100 µg/ml in the conventional method, the amount of antiserum necessary for attaining substantially the same curve as attained by the conventional method can be reduced to 1/18.

## Claims

1. An immunoassay for quantitatively determining an antigen, which comprises immobilizing a primary antibody onto the surface of a carrier via an affinity ligand, binding an antigen to be determined to the immobilized primary antibody, and reacting a labelled secondary antibody with said antigen.

2. An immunoassay of claim 1 wherein the affinity ligand is a substance capable of binding the Fc part in the antibody.

3. An immunoassay of claim 2 wherein the affinity ligand is protein A, protein G or anti-gamma IgG antibody.

4. An immunoassay of claim 2 wherein the affinity ligand is protein A.

5. An immunoassay of any of claims 1 to 4 wherein the primary antibody is IgG or antiserum.

6. An immunoassay of any of claims 1 to 5 wherein the antigen is a hormone, growth factor, bacterial toxin, bacterial metabolite or antibody thereto, exosporium component, virus capsid component, enzyme, lipoprotein, receptor, cancer marker, cell surface antigen, autoantibody, c-reactive protein or other physiological active substance.

7. An immunoassay of claim 6 wherein the hormone is growth hormone, insulin, adrenocorticotropic hormone (ACTH), thyroid-stimulating hormone (TSH), luteinizing hormone (LH) or gut hormone.

8. An immunoassay of claim 6 wherein the growth factor is epidermal growth factor (EGF), nerve growth factor (NGF), platelet-derived growth factor (PDGF), fibroblast growth factor (FGF), insulin-like growth factor (IGF) or hepatosite growth factor.

9. An immunoassay of claim 6 wherein the enzyme is alkaline phosphatase (ALP), glutamate-oxaloacetate transaminase (GOT), glutamate-pyruvate transaminase (GPT), lactate dehydrogenase (LDH), blood clotting factor, RNA polymerase or DNA polymerase.

10. An immunoassay of claim 6 wherein the said other physiological active substance is endothelin.
